Europäisches Patentamt

European Patent Office

Office européen des brevets

(11) Publication number: **0 432 479 A1**

## EUROPEAN PATENT APPLICATION

(21) Application number: 90121670.5

(22) Date of filing: 13.11.90

(51) Int. Cl.5 **A61K 9/48, A61K 9 66, A61K 47/10, A61K 37 30**

(30) Priority: 16.11.89 IT 2241089

(43) Date of publication of application:
19.06.91 Bulletin 91/25

(84) Designated Contracting States:
AT BE CH DE DK ES FR GB GR IT LI LU NL SE

(71) Applicant: SCLAVO S.p.A.
Via Fiorentina 1
I-53100 Siena(IT)

(72) Inventor: Lattanzi, Filippo
Via Sansedoni, 9
I-53100 Siena(IT)
Inventor: Vanni, Riccardo
Via Monte Santo, 3
I-53100 Siena(IT)

(74) Representative: Gervasi, Gemma et al
NOTARBARTOLO & GERVASI Srl Viale
Bianca Maria 33
I-20122 Milan(IT)

(54) Anhydrous pharmaceutical formulations and dosage forms for the rectal administration of calcitonin.

(57) New formulations are described containing a calcitonin as active principle and suitable for rectal administration in the form of soft gelatin capsules, in which the active principle, possibly mixed with a stabilizer, is dispersed in a liquid vehicle consisting of a polyethyleneglycol or a mixture of polyethyleneglycols of different molecular weight which has the consistency of a homogeneous fluid at ambient temperature, and possibly a $C_2$-$C_6$ alcohol with at least 2 hydroxyl groups, and present in a weight quantity of less than 15%. Said formulations, which contain no absorption adjuvant, enable the same pattern of hematic levels to be obtained as obtainable by conventional intramuscular administration, for equal unit dosages. Moreover, the absence of water makes said formulations highly stable.

EP 0 432 479 A1

# ANHYDROUS PHARMACEUTICAL FORMULATIONS AND DOSAGE FORMS FOR THE RECTAL ADMINISTRATION OF CALCITONIN

This invention relates to anhydrous pharmaceutical formulations suitable for rectal administration and containing a calcitonin as active principle.

Peptide compounds such as calcitonins are generally administered parenterally to man as the proteolytic enzymes present in the gastrointestinal tract rapidly hydrolyze them to oligopeptides devoid of pharmacological activity, they then being metabolized by the liver.

For these reasons the pharmaceutical forms in which calcitonins are commonly marketed are suitable for intramuscular or intravenous administration of the active principle.

In children and elderly people, who are the most frequent sufferers of hypercalcemial treatable by calcitonins, and especially in cases of long-term treatment, this creates considerable problems.

The possibility of administering such active principles rectally has therefore been the subject of numerous studies in recent years. This is because under normal conditions the drug does not degrade within the rectal ampulla but is adsorbed as such. In addition, as most of the adsorbed drug directly enters the blood circulation through the vena cava, thus bypassing the liver, it is not subjected to metabolic action by this organ.

Studies conducted on peptides and in particular on calcitonins have shown that such compounds are not easily adsorbed by the rectum walls. In addition, conventional rectal administration formulations containing calcitonins have shown poor hypercalcemic activity.

These results have led to the conclusion that an adsorption adjuvant has to be present in the formulation to obtain satisfactory hematic levels.

In this respect, considerable technical and patent literature exists regarding essentially the choice of particular adjuvants for the preparation of formulations suitable for rectal administration (JP-118013/81, JP-122309/81, NL-299/86 and EP-A-003743).

For example, European patent application EP-A-003743 describes the preparation of formulations for the rectal administration of peptide hormones and polysaccharides containing an adjuvant chosen from ascorbic acid and its salts, a salicylic acid derivative in which the hydroxyl is esterified, and an amino acid or a derivative thereof.

In particular, the examples relative to formulations containing a calcitonin as active principle show that:

a) in administering the claimed formulation, the dose required is five times the dose for intramuscular (i.m.) administration;

b) the use of other adjuvants chosen from those of the known art generally leads to worse results; and

c) in the absence of adjuvants, a dose equal to five times the dose for intramuscular administration does not give satisfactory results.

In order to overcome these drawbacks, other rectal calcitonin formulations have been proposed in the art.

For example, J. Pharm. Science 73(10), (1984), pp 13-66-1368, describes a formulation comprising calcitonin in a polyacrylic gel base.

Although the reproduced experimental data demonstrate that such a formulation significantly facilitates rectal adsorption of the peptide, they still show the need for doses of about 35 times the i.m. dose to obtain equivalent results.

In addition, the authors report that formulations containing calcitonin dispersed in polyethyleneglycol 1000 are not effective. From Italian patent application No. 22031 A/87 it is also known that it is possible to prepare a soft gelatin capsule for calcitonin rectal administration containing calcitonin, at least one polyethylene glycol, a $C_2$-$C_6$ alcohol and a physiologically acceptable aqueous buffer.

However the presence of water reduces the stability of the product. It has now been surprisingly found possible to prepare formulations based on calcitonin as active principle which are suitable inter alia for preparing rectal capsules, in the complete absence of an aqueous vehicle.

The patent application is based essentially on the discovery of the capacity of polyethyleneglycol to easily incorporate the active principle and possibly a stabilizer, both in lyophilized form.

It is therefore an object of the present invention to provide an anhydrous pharmaceutical formulation suitable for rectal administration, containing a calcitonin as active principle, a polyethyleneglycol or a mixture of polyethyleneglycols of different molecular weights, a $C_2$-$C_5$ alcohol but no aqueous vehicle.

A further object of the present invention is to provide unit dosage forms for rectal administration prepared using said formulation. Further objects of the present invention will be apparent from reading the following description and examples.

Specifically, the formulation of the present invention consists of a dispersion of the active principle, possibly mixed with a suitable stabilizer, both in anhydrous form in a vehicle which is fluid at ambient temperature, said vehicle being characterized by:

a) a polyethyleneglycol or a mixture of polyethyleneglycols of different molecular weight which has the consistency of a homogeneous fluid at ambient temperature and is present in a weight quantity exceeding 75% of the total weight of the formulation; and

b) a $C_2$-$C_6$ alcohol with at least 2 hydroxyl groups, and present in a weight quantity of less than 15% of the total weight of the formulation.

The formulation of the present invention is particularly useful for preparing soft gelatin capsules for use as unit dosages.

The capsules thus obtained, containing from 50 to 500 international units (IU) of calcitonin as active principle, are more stable with time.

With regard to the active principle for use in the formulation of the present invention, this can be chosen from calcitonins of natural or synthetic origin such as salmon calcitonin (SCT), eel calcitonin (ECT), pig calcitonin, or synthetic analogues such as $(ASu^{1-7})$ ECT commonly known as carbocalcitonin.

The quantity of active ingredient to be used in the formulation obviously depends on the desired content per unit dosage.

Generally, an active principle quantity of between 20 and 800 IU per ml of vehicle is used.

In certain cases, particularly when using eel calcitonin as active principle, it may be necessary to incorporate a stabilizer.

A particularly suitable stabilizer, as described in Italian patent application 20865 A.86 of the present applicant, is albumin and in particular human albumin, which has proved extremely effective both in increasing the intrinsic stability of calcitonin and in reducing its adsorption onto the walls of glass or plastics containers used in the preparation process.

If used, this stabilizer is contained in the formation in a quantity of between 0.01 and 1.2 mg and preferably between 0.05 and 1.0 mg per IU of calcitonin.

The polyethyleneglycol or mixture of polyethyleneglycols of different molecular weights for use as the base vehicle in the formulation of the present invention must be a homogeneous fluid at ambient temperature. Consequently polyethyleneglycols of low molecular weight, generally between 200 and 600, are used, possibly mixed with smaller quantities of higher molecular weight polyethyleneglycols in such proportions as to result in a homogeneous fluid vehicle at ambient temperature.

According to a preferred embodiment of the present invention, said polyethyleneglycol or mixtures of polyethyleneglycols of different molecular weights are used in a weight quantity exceeding 80% of the total formulation, and according to a still more preferred form in a quantity exceeding 85%.

Alcohols suitable for the purposes of the present invention are propylene glycol, ethylene glycol, sorbitol and, preferably, glycerin.

The alcohols, present as individual compounds or in mutual mixture, are present in the formulation in a weight quantity of less than 15%, and according to a particularly preferred form in a weight quantity of less than 10% of the total formulation.

The formulation of the present invention is therefore used to prepare soft gelatin capsules having in general a volumetric content of between 0.6 and 2.5 ml, operating in accordance with conventional methods known to the expert of the art, or in accordance with methods described in the technical and patent literature.

For example the solid components are melted and mixed with the other components in order to obtain an homogeneous mixture and then calcitonin is added to the above said mixture and the resulting mixture is homogenized and filled into soft gelatin capsules of a suitable unit dose size via a rotary die encapsulation machine or similar encapsulating device.

The gelatin casing can be prepared, for example, as described in patent applications EP 120248 and EP 121321.

The following examples illustrate in greater detail certain formulations and unit dosage forms representative of the present invention, together with the experimental results obtained on administering certain of these.

EXAMPLE 1

Soft gelatin capsules for rectal use provided by Scherer Company were filled with the following formulation expressed in terms of unit dosage (1):

| ECT | 100.00 IU |
|---|---|
| Polyethyleneglycol 600 | 850.00 mg |
| Polyethyleneglycol 4000 | 110.00 mg |
| Glycerin | 65.00 mg |
| Human albumin | 20.00 mg |
| Monobasic sodium phosphate monohydrate | 4.14 mg |

These capsules were administered to a group of 6 healthy volunteers, the hematic ECT being determined by RIA 30, 60 and 120 minutes after administration.

An equal dose (100 I.U.) of ECT was administered by i.m. injection to a further group of 6 healthy volunteers. Again in this case the hematic ECT was determined by RIA 30, 60 and 120 minutes after administration.

The mean results of the 6 volunteers of each group are shown in the following Table I

TABLE I

| Method of administration | Hematic ECT (pg/ml) | | |
|---|---|---|---|
| | 30 min | 60 min | 120 min |
| Rectal | 100 | 180 | 150 |
| Intramuscular | 120 | 250 | 180 |

EXAMPLE 2

Soft gelatin capsules for rectal use provided by Scherer Company were filled with the following formulation expressed in terms of unit dosage (2):

| ECT | 100 IU |
|---|---|
| Polyethyleneglycol 600 | 850.00 mg |
| Polyethyleneglycol 4000 | 110.00 mg |
| Glycerin | 55.00 mg |
| Anhydrous dibasic sodium phosphate | 2.30 mg |
| Citric acid monohydrate | 1.95 mg |
| Human albumin | 5.50 mg |

These capsules were administered to a group of 6 healthy volunteers, the hematic ECT being determined by RIA 30, 60 an 120 minutes after administration.

An equal dose (100 I.U.) of ECT was administered by i.m. injection to a further group of 6 healthy volunteers. Again in this case the hematic ECT was determined by RIA 30, 60 and 120 minutes after administration.

The mean results of the 6 volunteers of each group are shown in the following Table II.

TABLE II

| Method of administration | Hematic ECT (pg/ml) | | |
|---|---|---|---|
| | 30 min | 60 min | 120 min |
| Rectal | 90 | 200 | 150 |
| Intramuscular | 120 | 250 | 180 |

EXAMPLES 3-19

The following examples define formulations containing a calcitonin as active principle, suitable for rectal administration in the form of soft gelatin capsules.

The indicated doses refer to a formulation usable for the preparation of 1000 capsules.

| 3) ECT | 50,000 IU |
|---|---|
| Polyethyleneglycol 600 | 850.00 g |
| Polyethyleneglycol 4000 | 110.00 g |
| Glycerin | 65.00 g |
| Monobasic sodium phosphate monohydrate | 4.14 g |
| Human albumin | 20.00 g |

| 4) ECT | 150,000 IU |
|---|---|
| Polyethyleneglycol 600 | 850.00 g |
| Polyethyleneglycol 4000 | 110.00 g |
| Glycerin | 65.00 g |
| Monobasic sodium phosphate monohydrate | 4.14 g |
| Human albumin | 20.00 g |

| 5) ECT | 50,000 IU |
|---|---|
| Polyethyleneglycol 600 | 850.00 g |
| Polyethyleneglycol 4000 | 110.00 g |
| Glycerin | 55.00 g |
| Anhydrous dibasic sodium phosphate | 2.30 g |
| Citric acid monohydrate | 1.95 g |
| Human albumin | 5.50 g |

| 6) ECT | 150,000 IU |
|---|---|
| Polyethyleneglycol 600 | 850.00 g |
| Polyethyleneglycol 4000 | 110.00 g |
| Glycerin | 55.00 g |
| Anhydrous dibasic sodium phosphate | 2.30 g |
| Citric acid monohydrate | 1.95 g |
| Human albumin | 5.50 g |

| 7) ECT | 100,000 IU |
|---|---|
| Polyethyleneglycol 400 | 550.00 g |
| Glycerin | 50.00 g |

| 8) ECT | 100,000 IU |
|---|---|
| Polyethyleneglycol 200 | 550.00 g |
| Glycerin | 50.00 g |
| Human albumin | 5.00 g |

| 9) ECT | 100,000 IU |
|---|---|
| Polyethyleneglycol 200 | 450.00 g |
| Polyethyleneglycol 400 | 250.00 g |
| Polyethyleneglycol 4000 | 50.00 g |
| Glycerin | 40.00 g |
| Monobasic sodium phosphate monohydrate | 4.14 g |
| Human albumin | 20.00 g |

| 10) ECT | 150,000 IU |
|---|---|
| Polyethyleneglycol 200 | 450.00 g |
| Polyethyleneglycol 400 | 250.00 g |
| Polyethyleneglycol 4000 | 50.00 g |
| Glycerin | 40.00 g |
| Monobasic sodium phosphate monohydrate | 4.14 g |
| Human albumin | 20.00 g |

| 11) SCT | 100,000 IU |
|---|---|
| Polyethyleneglycol 200 | 450.00 g |
| Polyethyleneglycol 400 | 250.00 g |
| Polyethyleneglycol 4000 | 50.00 g |
| Glycerin | 40.00 g |
| Monobasic sodium phosphate monohydrate | 4.14 g |
| Human albumin | 20.00 g |

| 12) SCT | 100.00[24m |
|---|---|
| Polyethyleneglycol 600 | 850.00 g |
| Polyethyleneglycol 4000 | 110.00 g |
| Glycerin | 65.00 g |
| Monobasic sodium phosphate monohydrate | 4.14 g |
| Human albumin | 20.00 g |

| 13) SCT | 100,000 IU |
|---|---|
| Polyethyleneglycol 600 | 850.00 g |
| Polyethyleneglycol 4000 | 110.00 g |
| Glycerin | 65.00 g |
| Anhydrous dibasic sodium phosphate | 2.30 g |
| Citric acid monohydrate | 1.95 g |
| Human albumin | 5.50 g |

| 14) SCT | 150,000 IU |
|---|---|
| Polyethyleneglycol 600 | 850.00 g |
| Polyethyleneglycol 4000 | 110.00 g |
| Glycerin | 65.00 g |
| Monobasic sodium phosphate monohydrate | 4.14 g |
| Human albumin | 20.00 g |

| 15) SCT | 150,000 IU |
|---|---|
| Polyethyleneglycol 600 | 850.00 g |
| Polyethyleneglycol 4000 | 110.00 g |
| Glycerin | 65.00 g |
| Anhydrous dibasic sodium phosphate | 2.30 g |
| Citric acid monohydrate | 1.95 g |
| Human albumin | 5.50 g |

| 16) (ASU[1-7]) ECT | 150,000 IU |
|---|---|
| Polyethyleneglycol 600 | 850.00 g |
| Polyethyleneglycol 4000 | 110.00 g |
| Glycerin | 60.00 g |
| Anhydrous dibasic sodium phosphate | 2.30 g |
| Citric acid monohydrate | 1.95 g |
| Human albumin | 5.50 g |

EP 0 432 479 A1

| 17) (ASU$^{1-7}$) ECT | 100,000 IU |
|---|---|
| Polyethyleneglycol 600 | 850.00 g |
| Polyethyleneglycol 4000 | 110.00 g |
| Glycerin | 60.00 g |
| Monobasic sodium phosphate monohydrate | 4.14 g |
| Human albumin | 20.00 g |

| 18) (ASU$^{1-7}$) ECT | 100,000 IU |
|---|---|
| Polyethyleneglycol 600 | 850.00 g |
| Polyethyleneglycol 4000 | 110.00 g |
| Glycerin | 60.00 g |
| Anhydrous dibasic sodium phosphate | 2.30 g |
| Citric acid monohydrate | 1.95 g |
| Human albumin | 5.50 g |

| 19) (ASU$^{1-7}$) ECT | 150,000 IU |
|---|---|
| Polyethyleneglycol 600 | 850.00 g |
| Polyethyleneglycol 4000 | 110.00 g |
| Glycerin | 60.00 g |
| Monobasic sodium phosphate monohydrate | 4.14 g |
| Human albumin | 20.00 g |

**Claims**

1. An anhydrous pharmaceutical formulation containing a calcitonin as active principle and suitable for preparing capsules for rectal administration, characterised by containing, possibly mixed with a suitable stabilizer, a calcitonin dispersed in a fluid vehicle consisting of: a) polyethyleneglycol or a mixture of polyethyleneglycols of different molecular weight which has the consistency of a homogeneous fluid at ambient temperature and is present in a weight quantity exceeding 75% of the total weight of the formulation; and b) possibly a $C_2$-$C_6$ alcohol with at least two hydroxyl groups, and present in a weight quantity of less than 15% of the total formulation weight.

2. A pharmaceutical formulation as claimed in claim 1, wherein the polyethyleneglycol or mixture of polyethyleneglycols of different molecular weights is used in a weight quantity exceeding 80% of the total formulation weight.

3. A pharmaceutical formulation as claimed in claim 1, wherein the polyethyleneglycol or mixture of polyethyleneglycols of different molecular weights is used in a weight quantity exceeding 85% of the total formulation weight.

4. A pharmaceutical formulation as claimed in claim 1, wherein the alcohol possibly present in the fluid vehicle is chosen from propylene glycol, ethylene glycol, sorbitol, glycerin and mixtures thereof.

5. A pharmaceutical formulation as claimed in claim 1, wherein the alcohol possibly present is used in a weight quantity of less than 10% of the total formulation weight.

6. A pharmaceutical formulation as claimed in claim 1, wherein the stabilizer is albumin.

7. A pharmaceutical formulation as claimed in claim 6, wherein the albumin is present in a quantity of between 0.01 and 1.2 mg per I.U. of calcitonin.

8. A pharmaceutical formulation as claimed in claim 1, wherein the active principle is eel calcitonin.

9. A pharmaceutical formulation as claimed in claim 1, wherein the active principle is present in a quantity of between 200 and 800 I.U. per ml of formulation.

10. A unit dosage form suitable for rectal administration in the form of a soft gelatin capsule prepared using the formulation claimed in claims 1 to 9.

8

11. A unit dosage form as claimed in claim 10, containing from 50 to 500 I.U. of a calcitonin as active principle.

12. A soft gelatin capsule for the rectal administration of a calcitonin and containing:

a) from 50 to 500 I.U. of calcitonin possibly mixed with human albumin in a quantity of between 0.1 and 1.2 mg per I.U. of calcitonin;

b) a polyethyleneglycol or a mixture of polyethyleneglycols of different molecular weight which has the consistency of a homogeneous fluid at ambient temperature and is present in a weight quantity exceeding 80% of the total weight of the capsule contents;

c) a $C_2$-$C_6$ alcohol with at least two hydroxyl groups, and present in a weight quantity of less than 15% of the total weight of the capsule contents.

13. A capsule as claimed in claim 12, wherein the alcohol is glycerin.

Claims for the following Contracting States: ES, GR

1. Process for the preparation of an anhydrous pharmaceutical formulation containing a calcitonin as active principle and suitable for preparing capsules for rectal administration containing, possibly mixed with a suitable stabilizer, a calcitonin dispersed in a fluid vehicle consisting of : a) a polyethyleneglycol or a mixture of polyethyleneglycols of different molecular weight which has the consistency of a homogeneous fluid at ambient temperature and is present in a quantity exceeding 75% of the total weight of the formulation and b) possibly a $C_2$-$C_6$ alcohol with at least two hydroxyl groups, and present in a weight, quantity of less than 15% of the total formulation weight wherein the solid components are melted and mixed with the other components in order to obtain an homogeneous mixture and then calcitonin is added to the above said mixture and the resulting mixture is homogenized.

2. A process according to claim 1, wherein the polyethyleneglycol or mixture of polyethyleneglycols of different molecular weight is used in a weight quantity exceeding 80% of the total of the formulation weight.

3. A process according to claim 1, wherein the polyethyleneglycol or mixture of polyethyleneglycols of different molecular weight is used in a weight quantity exceeding 85% of the total of the formulation weight.

4. A process according to claim 1, wherein the alcohol possibly present in the fluid vehicle is chosen from propylene glycol, ethylene glycol, sorbitol, glycerin and mixtures thereof.

5. A process according to claim 1, wherein the alcohol possibly present is used in a weight quantity of less than 10% of the total formulation weight.

6. A process according to claim 1, wherein the stabilizer is albumin.

7. A process according to claim 6, wherein the albumin is present in a quantity of between 0.01 and 1.2 mg per I.U. of calcitonin.

8. A process according to claim 1, wherein the active principle is eel calcitonin.

9. A process according to claim 1, wherein the active principle is present in a quantity of between 200 and 800 I.U. per ml of formulation.

10. A process for the preparation of a unit dosage form suitable for rectal administration in the form of a soft gelatin capsule wherein a formulation prepared according to the process of claims 1 - 9 is filled into soft gelatine capsule.

11. A process according to claim 10 wherein the unit dosage form contains from 50 to 500 I.U. of a calcitonin as active principle.

12. A process for the preparation of a soft gelatin capsule for the rectal administration of a calcitonin containing :

a) from 50 to 500 I.U. of calcitonin possibly mixed with human albumin in a quantity of between 0.01 and 1.2 mg per I.U. of calcitonin

b) a polyethyleneglycol or a mixture of polyethyleneglycols of different molecular weight which has the consistency of a homogeneous fluid at ambient temperature and is present in a weight quantity exceeding 80% of the total weight of the capsule contents;

c) a $C_1$-$C_6$ alcohol with at least two hydroxyl groups, and present in a weight quantity of less than 15% of the total weight of the capsule contents

wherein the solid components are melted and mixed with the other components in order to obtain an homogeneous mixture and then calcitonin is added to the above said mixture and the resulting mixture is homogenized and filled into soft gelatin capsule.

European Patent
Office

EUROPEAN SEARCH REPORT

Application Number

EP 90 12 1670

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 308 725 (SCLAVO) <br> * Claims * <br> --- | 1-13 | A 61 K 9/48 <br> A 61 K 9/66 <br> A 61 K 47/10 <br> A 61 K 37/30 |
| A | EP-A-0 183 527 (YAMANOUCHI) <br> * Claim 1; page 2, lines 5-12,23-27; page 3, line 22 * <br> --- | 1,4,6,8 | |
| A | EP-A-0 225 189 (SCHERER) <br> * Claims 1,4,16,20-21 * <br> ----- | 1,4,8, 11 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 18-02-1991 | SCARPONI U. |

EPO FORM 1503 03.82 (P0401)